# EUROPÄISCHE PATENTSCHRIFT

(11) **EP 1 997 122 B1**
(45) Veröffentlichungstag und Bekanntmachung des Hinweises auf die Patenterteilung: **07.07.2010**
(21) Anmeldenummer: 07711471.8
(22) Anmeldetag: 09.02.2007
(51) Int. Cl.: H01J 33/00, A61L 2/00

(54) **VORRICHTUNG UND VERFAHREN ZUR EIGENSCHAFTSÄNDERUNG DREIDIMENSIONALER FORMTEILE MITTELS ELEKTRONEN**
DEVICE AND METHOD FOR ALTERING THE CHARACTERISTICS OF THREE-DIMENSIONAL SHAPED PARTS USING ELECTRONS
DISPOSITIF ET PROCÉDÉ DE MODIFICATION DES PROPRIÉTÉS D'UN CORPS MOULÉ TRIDIMENSIONNEL AU MOYEN D'ÉLECTRONS

(30) Priorität: 20.03.2006 DE 102006012666
(43) Veröffentlichungstag der Anmeldung: 03.12.2008
(73) Patentinhaber: Fraunhofer-Gesellschaft zur Förderung der angewandten Forschung e.V., 80686 München (DE)
(72) Erfinder: BARTEL, Rainer, 01324 Dresden (DE); GOHS, Uwe, Dr., 01169 Dresden (DE); KIRCHHOFF, Volker, Prof. Dr., 01324 Dresden (DE); MATTAUSCH, Gösta, Dr., 01454 Ullersdorf (DE); ROEDER, Olaf, Dr., 01326 Dresden (DE); KUBUSCH, Jörg, 01279 Dresden (DE)
(86) Internationale Anmeldenummer: PCT/EP2007/001096
(87) Internationale Veröffentlichungsnummer: WO 2007/107212

(56) Entgegenhaltungen:
- WO-A-94/28573
- WO-A-99/39750
- WO-A-99/39751

## Beschreibung

Die Erfindung betrifft eine Vorrichtung und ein Verfahren zum Modifizieren von Stoffeigenschaften an der Oberfläche, im Randbereich oder im gesamten Volumen dreidimensionaler Formteile mittels der Energie von Elektronen.

Mittels Elektronen kann Energie räumlich und zeitlich determiniert in Materialien eingetragen werden, um deren Stoffeigenschaften an der Oberfläche, in der Randschicht oder im Volumen zu verändern. Die dazu benötigten Elektronen werden in so genannten Elektronengeneratoren erzeugt, formiert und beschleunigt, ehe sie über ein meist ebenes Elektronenaustrittsfenster an einen Prozessraum abgegeben werden. Dabei ist die Elektronendichte über die gesamte Ausdehnung des Fensters annähernd konstant. Nach dem Durchdringen einer Gasschicht (zum Beispiel Luft) erreichen die Elektronen eine zu behandelnde Produktoberfläche.

Als Elektronengeneratoren werden Bandstrahler oder Axialstrahler eingesetzt. Ein als Axialstrahler ausgebildeter Elektronengenerator nach dem Stand der Technik umfasst zusätztich einen Elektronenstrahlablenkraum mit einem Strahlablenksystem, mittels dem ein erzeugter Elektronenstrahl periodisch über das gesamte Elektronenaustrittsfenster und im zeitlichen Mittel in allen Teilbereichen des Fensters mit annähernd gleicher Verweildauer abgelenkt wird.

Dreidimensionale Formteile, wie beispielsweise Kabel, Rohre, Verpackungen oder Stränge und Profile aus verschiedenen Materialien (zum Beispiel Kunststoffe, Papier) werden in unterschiedlichen Branchen (zum Beispiel Verpackungsindustrie, Pharmazie, Medizintechnik, Kunststoffindustrie) eingesetzt. Für bestimmte Anwendungen ist eine Eigenschaftsänderung (zum Beispiel Vernetzung, Härtung, Sterilisation) der gesamten Oberfläche, der Randschicht und/oder des Volumens des Formteiles erforderlich.

### Stand der Technik

Es ist bekannt, Eigenschaften der gesamten Oberfläche von dreidimensionalen Formteilen mittels Elektronenenergie zu beeinflussen, indem ein Formteil in mehreren Durchläufen und in veränderter Position an einem Elektronenaustrittsfenster vorbeigeführt wird. Bekannte Vorrichtungen zum Erzeugen von Elektronen für das Modifizieren von Formteileigenschaften sind derart gestaltet, dass über das gesamte Elektronenaustrittsfenster eine annähernd gleiche Elektronenenergiedichte abgegeben wird.

Durch das Verändern der Position eines Formteils wird sichergestellt, dass die gesamte Formteiloberfläche mit Elektronenenergie beaufschlagt wird. Ein Nachteil derartiger Vorrichtungen besteht darin, dass ein Mehrfachdurchlauf mit einem relativ hohen Zeitaufwand verbunden ist. Das Verändern der Position des Formteils zwischen den einzelnen Durchläufen kann auch nicht wahllos erfolgen, sondern muss derart abgestimmt sein, dass einzelne Oberflächenbereiche in der Summe nicht mit unterschiedlichen Elektronenenergiedichten beaufschlagt werden, was zu unterschiedlichen Eigenschaften führen würde.

Nach dem Stand der Technik wird die gesamte Oberfläche eines dreidimensionalen Formteils während nur eines Durchlaufs mittels Elektronenenergie modifiziert, indem mehrere (mindestens drei) Elektronenaustrittsfenster derart angeordnet werden, dass diese den Querschnitt des Formteils umschließen, wobei das Formteil zwischen diesen Elektronenaustrittsfenstern hindurchgeführt und somit über den gesamten Querschnittsumfang mit Elektronen beaufschlagt wird.

Von der Firma LINAC Technologies (Technische Beschreibung "ELECTRON BEAM SURFACE STERILISATION SYSTEM 200 KeV - The Ke VAC S") ist eine Vorrichtung zum Sterilisieren der Oberfläche von Formteilen mittels Elektronenenergie bekannt, bei der drei Elektronengeneratoren derart angeordnet sind, dass deren zugehörige Elektronenaustrittsfenster ein Volumen mit dem Querschnitt eines gleichschenkligen Dreiecks umschließen, durch das die zu sterilisierenden Formteile in einem Durchlauf hindurchgeführt werden. Mit derartigen Vorrichtungen wird zwar gegenüber bekannten Lösungen, bei denen ein Formteil in mehreren Durchläufen mit Elektronen beaufschlagt wird, der zeitliche Aufwand verringert, der technische Aufwand jedoch erheblich erhöht.

Es sind ähnliche Anordnungen von drei Elektronenaustrittsfenstern bekannt, bei denen die Elektronen jedoch nur mittels eines Generators erzeugt und mittels Ablenksystemen auf die drei Elektronenaustrittsfenster aufgeteilt werden.

Bei den bekannten Einrichtungen, bei denen drei und mehr Elektronenaustrittsfenster ein Formteil umschließen und bei denen über ein gesamtes Elektronenaustrittsfenster eine annähernd gleiche Elektronenenergiedichte abgegeben und ein Formteil nur in einem Durchlauf mit Elektronen beaufschlagt wird, können einzelne Oberflächenteilbereiche des Formteils in Abhängigkeit von dessen Geometrie und dem daraus resultierenden unterschiedlichen Abstand der Oberflächenteilbereiche von einem Elektronenaustrittsfenster mit einer unterschiedlichen Dosis (absorbierte Energie pro Masseeinheit) an Elektronenenergie beaufschlagt werden.

Um eine bestimmte Eigenschaft an einem Formteil zu realisieren, ist eine bestimmte Dosis an Elektronenenergie erforderlich. Zweckmäßigerweise wird die Leistung der Elektronenerzeuger derart eingestellt, dass an jenen Oberflächenbereichen, an denen die geringste Dosis ankommt, die dort ankommende Dosis genau oder mindestens der Dosis entspricht, die für das Modifizieren der Eigenschaft erforderlich ist. Alle anderen Oberflächenbereiche des Formteils werden zwangsläufig mit einer erhöhten Dosis beaufschlagt. Diese erhöhte Dosis an Energie wird auch als Überdosis bezeichnet. Je höher die Überdosis in einzelnen Bereichen eines Formteils ist, umso stärker weichen die Eigenschaften in diesen Bereichen von den Zielparametern ab. Eine Überdosis an Elektronenenergie wirkt sich jedoch nicht nur negativ auf die zu modifizierenden Eigenschaften eines Formteils aus, sondern kann auch zu unerwünschten oder gar prozessschädlichen Nebenwirkungen durch das Bilden unerwünschter Reaktionsprodukte (zum Beispiel Ozon) im Prozessgas (zum Beispiel Luft) führen.

Ein als Überdosisfaktor bezeichneter Parameter gibt an, um das Wievielfache eine erforderliche Dosis zum Einstellen einer gewünschten Eigenschaft überschritten wird. Mit den bekannten Einrichtungen werden in Abhängigkeit von der Geometrie zu modifizierender Formteile in einzelnen Oberflächenbereichen Überdosisfaktoren erreicht, die für viele Anwendungen nicht akzeptabel sind, um hinreichend gleichmäßige Eigenschaften über die gesamte Oberfläche zu realisieren und was auch die bereits genannten unerwünschten Nebenwirkungen mit sich bringt.

### Aufgabenstellung

Der Erfindung liegt daher das technische Problem zugrunde, eine Vorrichtung und ein Verfahren zu schaffen, mittels denen die Nachteile des Standes der Technik überwunden werden. Insbesondere sollen Vorrichtung und Verfahren geeignet sein, Stoffeigenschaften dreidimensionaler Formteile mit geringem zeitlichem Aufwand zu modifizieren. Dabei soll der Überdosisfaktor möglichst auf der gesamten Formteiloberfläche gering sein bzw. sollen gleichmäßige Stoffeigenschaftsänderungen auf der gesamten Oberfläche, Randschicht oder im gesamten Volumen der Formteile durchgeführt werden können.

Die Lösung des technischen Problems ergibt sich durch die Gegenstände mit den Merkmalen der Patentansprüche 1 und 13. Weitere vorteilhafte Ausgestaltungen der Erfindung ergeben sich aus den abhängigen Patentansprüchen.

Erfindungsgemäß umfasst eine Vorrichtung zur Stoffeigenschaftsänderung eines dreidimensionalen Formteils mittels Elektronen, umfassend mindestens einen Elektronengenerator zum Erzeugen beschleunigter Elektronen, mit denen die gesamte Oberfläche, Randschicht oder das gesamte Volumen des Formteils während eines Prozessdurchlaufs beaufschlagbar ist, und mindestens drei Elektronenaustrittsfenster, wobei die Elektronenaustrittsfenster derart angeordnet sind, dass diese den Querschnitt eines Prozessraumes weitgehend vollständig umschließen, wobei mittels einer ersten Einrichtung die über die Fläche mindestens eines ersten der mindestens drei Elektronenaustrittsfenster abgegebene Elektronenenergiedichte derart steuerbar ist, dass über einzelne Teilbereiche des mindestens einen ersten der mindestens drei Elektronenaustrittsfenster in Abhängigkeit von der Geometrie des Formteils oder/und der Position des Formteils innerhalb des Prozessraumes unterschiedliche Elektronenenergiedichten abgegeben werden.

Zum Erzeugen der beschleunigten Elektronen kann ein Elektronengenerator verwendet werden, von dem die Elektronen mit einer entsprechenden Umlenksteuerung auf zwei oder mehr Elektronenaustrittsfenster aufgeteilt werden. Alternativ kann jedoch auch jedem Elektronenaustrittsfenster ein separater Elektronengenerator zugeordnet sein. Als Elektronengenerator sind sowohl Bandstrahler als auch Axialstrahler sowie andere Einrichtungen, mit denen beschleunigte Elektronen generierbar sind, geeignet.

Mittels einer erfindungsgemäßen Vorrichtung kann beispielsweise in den Teilbereichen eines Elektronenaustrittsfensters, in denen dem Fenster Oberflächenbereiche eines Formteils mit großem Abstand gegenüberliegen, die Elektronenenergiedichte erhöht werden gegenüber Teilbereichen des Fensters, in denen dem Fenster Oberflächenbereiche des Formteils mit geringem Abstand gegenüberliegen, so dass möglichst alle Oberflächenbereiche des Formteils die gleiche Dosis absorbieren und somit über die gesamte Oberfläche in der zu modifizierenden Bearbeitungstiefe (Oberfläche, Randschicht oder das ganze Volumen) gleichmäßige Eigenschaften ausgebildet werden. Als Mittel zum Modifizieren der Elektronenenergiedichte über einzelne Teilbereiche eines Elektronenaustrittsfensters können beispielsweise zusätzliche magnetische oder/und elektrische Systeme verwendet werden, welche die Bewegungsrichtung der beschleunigten Elektronen beeinflussen.

Beim Verwenden von Axialstrahlern kann beispielsweise ein zusätzliches Magnetsystem eingesetzt werden, das beim Scannen des Elektronenstrahls über ein Elektronenaustrittsfenster die zeitlich gemittelte Verweildauer des Elektronenstrahls in einzelnen Teilbereichen des Elektronenaustrittsfensters unterschiedlich verlängert oder verkürzt und somit die Elektronenenergiedichte über den Teilbereichen des Elektronenaustrittsfensters erhöht oder verringert.

Neben eben geformten Elektronenaustrittsfenstern können einzelne oder alle Elektronenaustrittsfenster beispielsweise auch zum Formteil hin konkav ausgebildet oder der Geometrie des Formteils angepasst sein, was bewirkt, dass möglichst viele Oberflächenabschnitte eines Formteils mit annähernd gleichem Maß vom gegenüberliegenden Elektronenaustrittsfenster beabstandet sind, wodurch geringere Überdosisfaktoren erzielt bzw. gleichmäßigere Eigenschaftsmodifikationen durchgeführt werden können. Die Anzahl der Elektronenaustrittsfenster einer erfindungsgemäßen Vorrichtung ist nach oben hin unbegrenzt.

Vorteilhaft wirkt sich auch ein Sensorsystem aus, welches erfasst, ob sich ein Formteil im Prozessraum befindet und welches in Abhängigkeit davon das Erzeugen von Elektronen steuert, so dass die Leistung der Elektronengeneratoren beispielsweise dann, wenn sich ein Formteil im Prozessraum befindet, auf einen prozessspezifischen Wert eingestellt wird und anderenfalls abgesenkt bzw. ganz auf Null reduziert wird.

Eine weitere Ausführungsform der Erfindung zeichnet sich dadurch aus, dass mindestens ein Elektronenaustrittsfenster bewegbar angeordnet ist. So kann dieses Elektronenaustrittsfenster beispielsweise zu Beginn, wenn ein Formteil in den Prozessraum eingebracht wird, zur Stirnseite des Formteils hin verkippt werden, um stirnseitig das Beaufschlagen mit Elektronen zu verbessern. Beim weiteren Transport des Formteils durch den Prozessraum hindurch kann das Fenster dann in Richtung ursprünglicher Ausrichtung und beim Verlassen des Prozessraumes in Richtung Rückseite des Formteiles verkippt werden. Es ist jedoch auch möglich, dass mit einem Fenster andere Bewegungsformen ausgeführt werden. So kann das Fenster beispielsweise zeitweise in Bewegungsrichtung des Formteils mitgeführt werden.

Um festzustellen, ob alle Oberflächenbereiche eines Formteils mit annähernd der gleichen Elektronendosis beaufschlagt werden, kann eine Messeinrichtung eingesetzt werden, die in verschiedenen, für das Beaufschlagen eines Formteils relevanten, Teilbereichen des Prozessraumes Werte erfasst, die die Elektronenenergiedichte charakterisieren. In Abhängigkeit von diesen Messergebnissen kann dann beispielsweise die über ein Elektronenaustrittsfenster abgegebene Elektronenenergiedichteverteilung gesteuert oder/und ein Elektronenaustrittsfenster bewegt werden.

Eine weitere Optimierung bei der Zielstellung, die Eigenschaften gleichmäßig über die gesamte Oberfläche eines Formteils zu modifizieren, ist mittels einer Einrichtung möglich, die über magnetische oder/und elektrische Ablenksysteme nicht nur den Punkt steuert, an dem ein Elektron ein Elektronenaustrittsfenster verlässt, sondern auch die Austrittsrichtung des Elektrons an diesem Punkt. Damit lassen sich noch gezielter bestimmte Oberflächenbereiche des Formteils mit Elektronen beaufschlagen.

Bei einer bevorzugten Ausführungsform ist zumindest ein Elektronenaustrittsfenster als vakuumdichte Folie und somit als Druckbarriere zwischen Strahlführungsraum und Prozessraum ausgebildet. Alternativ kann ein Elektronenaustrittsfenster auch als gasdurchlässige Druckstufenanordnung zwischen Elektronengenerator und Prozessraum ausgebildet sein.

Vorhergehend.wurden erfindungsgemäße Vorrichtungen nur dahingehend beschrieben, dass mit diesen annähernd gleiche Eigenschaften in allen Teilbereichen eines Formteils umsetzbar sind. Eine erfindungsgemäße Vorrichtung ist jedoch auch durch die in Teilbereichen separat steuerbare Elektronenenergiedichte dafür geeignet, ein Formteil in Teilbereichen mit unterschiedlichen Stoffeigenschaften auszustatten, wenn dies eine Aufgabenstellung fordert.

Erfindungsgemäße Vorrichtungen können beispielsweise zum Modifizieren der Stoffeigenschaften von Kunststoffformteilen (beispielsweise Kabel, Rohe, Schläuche, Profile, Stränge), Sterilisieren von Ausgangs-, Zwischen- oder Endprodukten der Pharmaindustrie, Sterilisieren von Verpackungen (beispielsweise aus Kunststoff, Pappe oder Papier) oder Desinfizieren von Gegenständen verwendet werden.

Bei einem erfindungsgemäßen Verfahren zum Ändern der Stoffeigenschaften eines dreidimensionalen Formteils mittels Elektronen erzeugt mindestens ein Elektronengenerator Elektronen, die derart durch mindestens drei Elektronenaustrittsfenster, die den Querschnitt eines Prozessraumes weitgehend vollständig umschließen, beschleunigt werden, dass die Elektronen die gesamte Oberfläche, Randschicht oder das gesamte Volumen des Formteils während einer Durchquerung des Prozessraumes beaufschlagen, wobei mittels einer ersten Einrichtung die über die Fläche mindestens eines ersten des mindestens drei Eektronenaustrittsfenster abgegebene Elektronenenergiedichte derart gesteuert wird, dass über einzelne Teilbereiche des mindestens einen ersten der mindestens drei Elektronenaustrittsfenster in Abhängigkeit von der Geometrie des Formteils oder/und der Position des Formteils im Prozessraum unterschiedliche Elektronenenergiedichten abgegeben werden.

Bei einer vorteilhaften Ausführungsform werden mittels einer Messeinrichtung während der Elektronenbehandlung Werte erfasst, die die Elektronenenergiedichte in verschiedenen Teilbereichen des Prozessraumes charakterisieren. In Abhängigkeit von den erfassten Werten kann dann beispielsweise mittels eines Regelkreises die über einzelne Teilbereiche des mindestens einen ersten der mindestens drei Elektronenaustrittsfenster abgegebene Elektronenenergiedichte separat gesteuert werden. Dadurch kann sichergestellt werden, dass an einem Teilbereich der Formteiloberfläche auch die dafür vorgesehene Elektronenenergiemenge ankommt. Veränderte Elektronenenergiedichteverhältnisse innerhalb eines Prozessraumes während der Lebensdauer einer Behandlungseinrichtung beispielsweise aufgrund von Alterungsprozessen der Einrichtung, Verunreinigungen eines Elektronenaustrittsfensters oder anderen Prozessschwankungen können durch einen derartigen Regelkreis ausgeglichen werden.

### Ausführungsbeispiel

Die Erfindung wird nachfolgend anhand eines bevorzugten Ausführungsbeispiels näher erläutert. Die einzige Figur zeigt eine schematische Querschnittsdarstellung einer erfindungsgemäßen Vorrichtung.

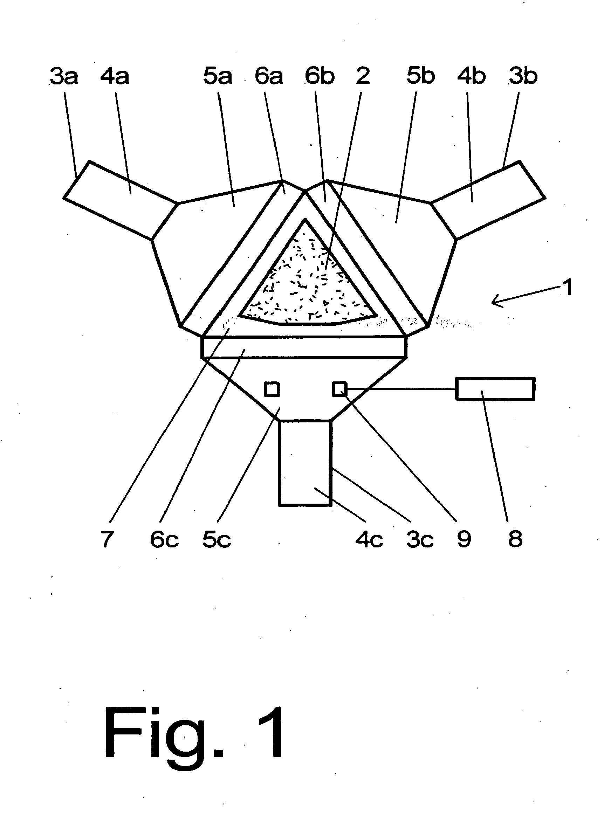

In Fig. 1 ist schematisch eine Vorrichtung 1 zur Elektronenbehandlung zum Zwecke der Vernetzung der gesamten Oberflächenschicht eines Formteils 2 im Querschnitt dargestellt. Vorrichtung 1 besteht aus drei als Axialstrahler ausgebildeten Elektronenerzeugern 3a, 3b, 3c, die jeweils einen Elektronengenerator 4a, 4b, 4c mit Strahlführungsraum 5a, 5b, 5c und ein als vakuumdichte Folie ausgebildetes Elektronenaustrittsfenster 6a, 6b, 6c umfassen. Die drei Elektronenerzeuger sind derart zueinander ausgerichtet, dass deren Elektronenaustrittsfenster den dreieckigen Querschnitt eines Prozessraumes 7 vollständig umschließen. Formteil 2, welches den Querschnitt eines unregelmäßigen Fünfecks aufweist, wird mittels eines nicht dargestellten Transportsystems während der Elektronenbehandlung durch den Prozessraum 7 geführt.

Elektronenerzeuger 3c umfasst des Weiteren eine Steuereinrichtung 8, die mit einem zusätzlichen Ablenksystem 9 in Form eines Magnetspulensystems innerhalb des Strahlführungsraumes 5c gekoppelt ist. Steuereinrichtung 8 und Ablenksystem 9 ermöglichen das unterschiedliche Verändern der über das zeitliche Mittel betrachteten Verweilzeit des von Elektronengenerator 4c erzeugten Elektronenstrahls in verschiedenen Teilbereichen über dem Elektronenaustrittsfenster 6c. Dabei wird die Verweildauer des Elektronenstrahls in einem Teilbereich des Fensters 6c umso mehr verlängert je weiter der Oberflächenbereich des Formteils 2, der dem Teilbereich des Fensters 6c gegenüberliegt, entfernt ist. Beim Elektronenaustrittsfenster 6c trifft dies auf die beiden Seitenbereiche des Fensters zu, die den zum Fenster 6c nicht parallel verlaufenden Seiten des Formteils 2 gegenüberliegen.

Auf diese Weise lässt sich die über die Fläche des Elektronenaustrittsfensters 6c abgegebene Elektronenergiedichte in einzelnen Teilbereichen separat derart.steuern, dass annähernd alle Teilbereiche der Oberfläche des Formteils 2, die dem Elektronenaustrittsfenster 6c gegenüberliegen, die gleiche Dosis Elektronenenergie absorbieren und somit nach dem Modifizieren annähernd gleiche Stoffeigenschaften aufweisen.

Bei den Elektronenerzeugern 3a und 3b ist eine derartige zusätzliche Ablenksteuerung für die Elektronen nicht vorhanden, weil alle Oberflächenbereiche des Formteils 2, die den Elektronenaustrittsfenstern der Elektronenerzeuger 3a und 3b gegenüberliegen, parallel zu diesen ausgerichtet sind und eine separate Steuerung der Elektronenenergiedichte in Teilbereichen über diesen Fenstern somit nicht erforderlich ist.

## Patentansprüche

1. Vorrichtung zur Stoffeigenschaftsänderung eines dreidimensionalen Formteils (2) mittels Elektronen, umfassend mindestens einen Elektronengenerator (4a; 4b; 4c) zum Erzeugen beschleunigter Elektronen, mit denen die gesamte Oberfläche, Randschicht oder das gesamte Volumen des Formteils (2) während eines Prozessdurchlaufs beaufschlagbar ist, und mindestens drei Elektronenaustrittsfenster (6a; 6b; 6c), wobei die Elektronenaustrittsfenster derart angeordnet sind, dass diese den Querschnitt eines Prozessraumes (7) weitgehend vollständig umschließen, **dadurch gekennzeichnet, dass** mittels einer ersten Einrichtung (8; 9) die über die Fläche mindestens eines ersten der mindestens drei Elektronenaustrittsfenster (6c) abgegebene Elektronenenergiedichte derart steuerbar ist, dass über einzelne Teilbereiche des mindestens einen ersten der mindestens drei Elektronenaustrittsfenster (6c) in Abhängigkeit von der Geometrie des Formteils (2) oder/und der Position des Formteils innerhalb des Prozessraumes (7) unterschiedliche Elektronenenergiedichten abgegeben werden.

2. Vorrichtung nach Anspruch 1, **dadurch gekennzeichnet, dass** die Flächen der Elektronenaustrittsfenster (6a; 6b; 6c) eben ausgebildet sind.

3. Vorrichtung nach Anspruch 1, **dadurch gekennzeichnet, dass** die Fläche mindestens eines Elektronenaustrittsfensters zum Formteil hin konkav ausgebildet ist.

4. Vorrichtung nach Anspruch 1 oder 3, **dadurch gekennzeichnet, dass** die Fläche mindestens eines Elektronenaustrittsfensters der Geometrie des Formteils angepasst ist.

5. Vorrichtung nach einem der vorhergehenden Ansprüche, **gekennzeichnet durch** mindestens eine Messeinrichtung, mittels der während der Elektronenbehandlung Werte erfassbar sind, die die Elektronenenergiedichte in verschiedenen Teilbereichen des Prozessraumes charakterisieren.

6. Vorrichtung nach einem der vorhergehenden Ansprüche, **dadurch gekennzeichnet, dass** mindestens ein Elektronenaustrittsfenster in Abhängigkeit von der Geometrie des Formteils oder/und der Position des Formteils im Prozessraum bewegbar ist.

7. Vorrichtung nach einem der vorhergehenden Ansprüche, **dadurch gekennzeichnet, dass** mindestens ein Elektronenaustrittsfenster (6a; 6b; 6c) als vakuumdichte Folie ausgebildet ist.

8. Vorrichtung nach einem der vorhergehenden Ansprüche, **dadurch gekennzeichnet, dass** mindestens ein Elektronenaustrittsfenster als gasdurchlässige Druckstufenanordnung zwischen Elektronengenerator und Prozessraum ausgebildet ist.

9. Vorrichtung nach einem der vorhergehenden Ansprüche, **gekennzeichnet durch** ein Sensorsystem, mittels dem die Leistung der Elektronenerzeuger in Abhängigkeit davon, ob sich ein Formteil im Prozessraum befindet, auf einen prozessspezifischen Wert einstellbar ist.

10. Vorrichtung nach einem der vorhergehenden Ansprüche, **gekennzeichnet durch** eine zweite Einrichtung, mittels der die Austrittsrichtung eines Elektrons aus einem Elektronenaustrittsfenster steuerbar ist.

11. Vorrichtung nach einem der vorhergehenden Ansprüche, **dadurch gekennzeichnet, dass** der mindestens eine Elektronengenerator als Bandstrahler oder Axialstrahler ausgebildet ist.

12. Verwendung einer Vorrichtung nach einem der vorhergehenden Ansprüche zum Modifizieren von Kunststoffen, Sterilisieren von Ausgangs-, Zwischen- und Endprodukten der Pharmaindustrie, Sterilisieren von Verpackungen oder Desinfizieren von Gegenständen.

13. Verfahren zum Ändern der Stoffeigenschaften eines dreidimensionalen Formteils (2) mittels Elektronen, wobei mindestens ein Elektronengenerator (4a; 4b; 4c) Elektronen erzeugt und derart durch mindestens drei Elektronenaustrittsfenster (6a; 6b; 6c), die den Querschnitt eines Prozessraumes (7) weitgehend vollständig umschließen, beschleunigt, dass die Elektronen die gesamte Oberfläche, Randschicht oder das gesamte Volumen des Formteils (2) während einer Durchquerung des Prozessraumes (7) beaufschlagen, **dadurch gekennzeichnet, dass** mittels einer ersten Einrichtung (8; 9) die über die Fläche mindestens eines ersten der mindestens drei Elektronenaustrittsfenster (6c) abgegebene Elektronenenergiedichte derart gesteuert wird, dass über einzelne Teilbereiche des mindestens einen ersten der mindens drei Elektronenaustrittsfenster (6c) in Abhängigkeit von der Geometrie des Formteils (2) oder/und der Position des Formteils (2) im Prozessraum (7) unterschiedliche Elektronenenergiedichten abgegeben werden.

14. Verfahren nach Anspruch 13, **dadurch gekennzeichnet, dass** mittels mindestens einer Messeinrichtung während der Elektronenbehandlung Werte erfasst werden, die die Elektronenenergiedichte in verschiedenen Teilbereichen des Prozessraumes charakterisieren.

15. Verfahren nach Anspruch 14, **dadurch gekennzeichnet, dass** in Abhängigkeit der erfassten Werte mittels eines Regelkreises die über einzelne Teilbereiche des mindestens einen ersten der mindestens drei Elektronenaustrittsfenster abgegebene Elektronenenergiedichte separat gesteuert wird.

## Claims

1. Apparatus for altering the material properties of a three-dimensional shaped part (2) by means of electrons, comprising at least one electron generator (4a; 4b; 4c) for generating accelerated electrons, with which the entire surface, outer layer or entire volume of the shaped part (2) can be bombarded during a process run, and at least three electron exit windows (6a; 6b; 6c), the electron exit windows being arranged in such a way that they enclose the cross section of a process chamber (7) largely completely, **characterized in that,** by means of a first device (8; 9), the electron energy density emitted over the surface area of at least a first of the at least three electron exit windows (6c) can be controlled in such a way that different electron energy densities are emitted over individual partial regions of at least the first of the at least three electron exit windows (6c), in dependence on the geometry of the shaped part (2) and/or the position of the shaped part within the process chamber (7).

2. Apparatus according to Claim 1, **characterized in that** the surface areas of the electron exit windows (6a; 6b; 6c) are of a planar form.

3. Apparatus according to Claim 1, **characterized in that** the surface area of at least one electron exit window is of a concave form towards the shaped part.

4. Apparatus according to Claim 1 or 3, **characterized in that** the surface area of at least one electron exit window is adapted to the geometry of the shaped part.

5. Apparatus according to one of the preceding claims, **characterized by** at least one measuring device, by means of which values which characterize the electron energy density in various partial regions of the process chamber can be detected during the electron treatment.

6. Apparatus according to one of the preceding claims, **characterized in that** at least one electron exit window is movable, in dependence on the geometry of the shaped part and/or the position of the shaped part in the process chamber.

7. Apparatus according to one of the preceding claims, **characterized in that** at least one electron exit window (6a; 6b; 6c) is formed as a vacuum-tight film.

8. Apparatus according to one of the preceding claims, **characterized in that** at least one electron exit window is formed as a gas-permeable pressure stage arrangement between the electron generator and the process chamber.

9. Apparatus according to one of the preceding claims, **characterized by** a sensor system, by means of which the power of the electron generator can be set to a process-specific value in a dependence on whether there is a shaped part in the process chamber.

10. Apparatus according to one of the preceding claims, **characterized by** a second device, by means of which the exiting direction of an electron from an electron exit window can be controlled.

11. Apparatus according to one of the preceding claims, **characterized in that** the at least one electron generator is formed as a band emitter or an axial emitter.

12. Use of an apparatus according to one of the preceding claims for modifying plastics, sterilizing raw, intermediate and end products of the pharmaceuticals industry, sterilizing packagings or disinfecting articles.

13. Method for altering the material properties of a three-dimensional shaped part (2) by means of electrons, at least one electron generator (4a; 4b; 4c) generating electrons and accelerating them through at least three electron exit windows (6a; 6b; 6c), which enclose the cross section of a process chamber (7) largely completely, in such a way that the electrons bombard the entire surface, outer layer or entire volume of the shaped part (2) during a pass across the process chamber (7), **characterized in that**, by means of a first device (8; 9), the electron energy density emitted over the surface area of at least a first of the at least three electron exit windows (6c) can be controlled in such a way that different electron energy densities are emitted over individual partial regions of at least a first of the at least three electron exit windows (6c), in dependence on the geometry of the shaped part (2) and/or the position of the shaped part (2) within the process chamber (7).

14. Method according to Claim 13, **characterized in that** values which characterize the electron energy density in various partial regions of the process chamber are detected by means of at least one measuring device during the electron treatment.

15. Method according to Claim 14, **characterized in that** the electron energy density emitted over individual partial regions of at least a first of the at least three electron exit windows is separately controlled in dependence on the detected values by means of a closed-loop control circuit.

## Revendications

1. Dispositif pour modifier les propriétés de la matière d'une pièce moulée tridimensionnelle (2) par des électrons, dispositif comprenant au moins un générateur d'électrons (4a, 4b, 4c) pour générer des électrons accélérés appliqués à toute la surface, à la couche marginale et à l'ensemble du volume de la pièce moulée (2) au cours du procédé et au moins trois fenêtres de sortie d'électrons (6a, 6b, 6c),
les fenêtres de sortie d'électrons étant installées de façon à entourer de manière pratiquement complète la section d'une chambre de procédé (7),
**caractérisé en ce qu'**
à l'aide d'une première installation (8, 9), on commande au moins une première des trois fenêtres de sortie d'électrons (6c) pour la densité d'énergie fournie par les électrons de façon qu'on obtienne des densités différentes d'énergie d'électrons, par des zones partielles séparées d'au moins la première des trois fenêtres de sortie d'électrons (6c), en fonction de la géométrie de la pièce moulée (2) et/ou de la position de la pièce moulée (2) dans la chambre de procédé (7).

2. Dispositif selon la revendication 1,
**caractérisé en ce que**
les surfaces des fenêtres de sortie d'électrons (6a, 6b, 6c), sont de forme plane.

3. Dispositif selon la revendication 1,
**caractérisé en ce que**
la surface d'au moins une fenêtre de sortie d'électrons, est concave en direction de la pièce moulée.

4. Dispositif selon les revendications 1 ou 3,
**caractérisé en ce que**
la surface d'au moins une fenêtre de sortie d'électrons, est adaptée à la géométrie de la pièce moulée.

5. Dispositif selon l'une des revendications précédentes,
**caractérisé par**
au moins une installation de mesure permettant de saisir les valeurs pendant le traitement par les électrons, valeurs caractérisant la densité de l'énergie des électrons dans les différentes zones partielles de la chambre de procédé.

6. Dispositif selon l'une des revendications précédentes,
**caractérisé en ce qu'**
au moins une fenêtre de sortie d'électrons peut être déplacée en fonction de la géométrie de la pièce moulée et/ou de la position de la pièce moulée dans la chambre de procédé.

7. Dispositif selon l'une des revendications précédentes,
**caractérisé en ce qu'**
au moins une fenêtre de sortie d'électrons (6a, 6b, 6c) est réalisée sous la forme d'un film étanche au vide.

8. Dispositif selon l'une des revendications précédentes,
**caractérisé en ce qu'**
au moins une fenêtre de sortie d'électrons est réalisée comme montage d'étage de pression perméable aux gaz, entre le générateur d'électrons et la chambre de procédé.

9. Dispositif selon l'une des revendications précédentes,
**caractérisé par**
un système de capteurs permettant de régler la puissance du générateur d'électrons sur une valeur spécifique au procédé, en fonction de la présence d'une pièce moulée, dans la chambre de procédé.

10. Dispositif selon l'une des revendications précédentes,
**caractérisé par**
une seconde installation permettant de commander la direction de sortie d'un électron à travers la fenêtre de sortie d'électrons.

11. Dispositif selon l'une des revendications précédentes,
**caractérisé en ce qu'**
au moins un générateur d'électrons est réalisé sous la forme d'un dispositif d'émission selon une bande ou d'un dispositif d'émission axial.

12. Application d'un dispositif selon l'une des revendications précédentes pour modifier des matières plastiques, stériliser des matières premières, des produits intermédiaires ou des produits finis, de l'industrie pharmaceutique, pour stériliser des emballages ou désinfecter des objets.

13. Procédé pour modifier les propriétés de la matière d'une pièce moulée tridimensionnelle (2) par des électrons, dispositif comprenant au moins un générateur d'électrons (4a, 4b, 4c) pour générer des électrons accélérés, appliqués à toute la surface, à la couche marginale et à l'ensemble du volume de la pièce moulée (2) au cours du procédé et au moins trois fenêtres de sortie d'électrons (6a, 6b, 6c),
les fenêtres de sortie d'électrons étant installées de façon à entourer de manière pratiquement complète, la section d'une chambre de procédé (7),
**caractérisé en ce qu'**
à l'aide d'une première installation (8, 9), on commande au moins une première des trois fenêtres de sortie d'électrons (6c) pour la densité d'énergie fournie par les électrons de façon qu'on obtienne des densités différentes d'énergie d'électrons, par des zones partielles séparées d'au moins la première des trois fenêtres de sortie d'électrons (6c), en fonction de la géométrie de la pièce moulée (2) et/ou de la position de la pièce moulée (2) dans la chambre de procédé (7).

14. Procédé selon la revendication 13,
**caractérisé en ce qu'**
à l'aide d'au moins une installation de mesure, on saisit pendant le traitement par les électrons, des valeurs qui représentent la densité de l'énergie des électrons dans les différentes zones partielles de la chambre du procédé.

15. Procédé selon la revendication 14,
**caractérisé en ce qu'**
en fonction des valeurs saisies, à l'aide d'un circuit de régulation, on commande séparément la densité d'énergie des électrons fournis à travers la fenêtre de sortie des électrons dans les différentes zones partielles d'au moins une première des trois fenêtres de sortie d'électrons.
